(19) **Europäisches Patentamt**

**European Patent Office**

**Office européen des brevets**

(11) **EP 1 305 248 B1**

(12) **EUROPEAN PATENT SPECIFICATION**

(45) Date of publication and mention
of the grant of the patent:
**04.01.2006 Bulletin 2006/01**

(51) Int Cl.:
**B65H 51/04** (2006.01)

(21) Application number: **01942069.4**

(22) Date of filing: **07.06.2001**

(86) International application number:
**PCT/US2001/018469**

(87) International publication number:
**WO 2001/094247 (13.12.2001 Gazette 2001/50)**

(54) **APPARATUS AND METHODS FOR FEEDING YARN**

FADENLIEFERVORRICHTUNG UND -VERFAHREN

DISPOSITIF ET PROCEDE D'APPORT DE FIL

(84) Designated Contracting States:
**DE FR GB IT TR**

(30) Priority: **08.06.2000 JP 2000171461**

(43) Date of publication of application:
**02.05.2003 Bulletin 2003/18**

(73) Proprietor: **DuPont-Toray Co., Ltd.**
**Tokyo 103-0027 (JP)**

(72) Inventors:
• **HASHIMOTO, Takeshi**
**Kourachou 522-0261 (JP)**
• **HAYASHI, Shoichi**
**Ohtsu 520-2142 (JP)**
• **TANAKA, Hiroko**
**Ashiyashi 659-0015 (JP)**

(74) Representative: **Thomson, James B.**
**Frank B. Dehn & Co.**
**179 Queen Victoria Street**
**London EC4V 4EL (GB)**

(56) References cited:
EP-A- 0 945 534        US-A- 1 922 146
US-A- 3 099 143        US-A- 3 209 558
US-A- 3 279 670        US-A- 3 774 829
US-A- 3 912 185

• **"37922 STRAND CONTROL"**, RESEARCH DISCLOSURE, KENNETH MASON PUBLICATIONS, HAMPSHIRE, GB, NR. 379, PAGE(S) 729-730 XP000549189 ISSN: 0374-4353 the whole document
• PATENT ABSTRACTS OF JAPAN vol. 1995, no. 01, 28 February 1995 (1995-02-28) & JP 06 298453 A (ASAHI CHEM IND CO LTD), 25 October 1994 (1994-10-25)

## Description

This invention relates to an apparatus and methods for feeding fiber to downstream equipment and, more specifically, to feeding elastomeric fibers (US-A-3 099 143).

## Description of Background Art

When elastic combination yarns, stretch fabrics, and stretch laminates are manufactured from natural, semi-synthetic, or synthetic fibers, the elastomeric fiber is typically removed from its wound package by a rolling takeoff mode. In this process, the package is rotated, and the fiber is fed to downstream equipment at a speed faster than the rotational speed of the package in an attempt to maintain constant tension. Even so, the tension can be uneven, which can result in nonuniformities in the products made from these fibers. These difficulties are especially serious for elastomeric fibers such as spandex, particularly when no lubricating finish is used, for example in making disposable diapers and sanitary napkins. Further, because the package is rotated, transfer tails cannot be used, and when a package has been emptied, the downstream operation must be stopped, a new package mounted, and the end of the old package tied to the beginning of a new package. This lowers the operating efficiency of the operation and is particularly costly for the high-speed diaper lines in use today.

Alternatively, transfer tails can be used with over-end-take-off (OETO), in which the fiber is pulled from a stationary package over the end of the tubecore on which the fiber has been wound. However, the problem of nonuniform tension is especially acute with OETO.

Feeders for improving the uniformity of the tension while fibers are being unwound from packages have been disclosed in United States Patents 4,953,367 and 6,105,398 (corresponding of EP-A-0 945 534), but such feeders are complex and expensive, and their durability can be inadequate when high decitex fibers and high tensions are used. Use of a conventional, fixed-speed roll as a feed roll can result in roll-wraps and fiber breaks when the fiber friction is high, even if the roll and/or the fiber have a matte finish.

Furthermore, when a synthetic fiber, especially one having a high coefficient of friction and high elongation, is unwound from a package, it can be difficult to feed the yarn with uniform tension from all layers in the package. This is particularly true for an elastomeric fiber wound onto a cylindrical tube to a package weight of more than 1 kg, because in this case the unwinding tension can vary considerably from one layer to another within the package, due to package relaxation. Conventional correction of package relaxation requires complex equipment such as that described in British Patent GB 1,219,395 or electronic equivalents of such equipment.

Changes in the average unwinding tension and the coefficient of variation in tension can result in feeding yarn of differing length and size to a downstream operation which, in turn, can lead to unacceptable nonuniformities in the final product.

A simple apparatus for maintaining uniform tension and correcting nonuniform package relaxation, while reliably and rapidly feeding fibers to downstream equipment, is still needed.

## SUMMARY OF THE INVENTION

The present invention provides a compound roll for feeding at least one fiber to downstream equipment, characterised in that said compound roll comprises a plurality of undriven first rolls wherein each first roll has an axis and is freely rotatably mounted on a second, driven rotatable roll which has a diameter larger than the diameter of each first roll and an axis, wherein the axis of each first roll is substantially parallel to the axis of the second roll, and the axes of the plurality of first rolls are substantially radially symmetrically arranged around the axis of the second roll.

## BRIEF DESCRIPTION OF THE FIGURES

FIG. 1 is a schematic perspective view showing an embodiment of the compound roll of the invention.
FIG's. 2, 3, and 4 are schematic end views showing other embodiments of the compound roll of the invention.
FIG. 5 is a schematic showing of a compound roll of the invention in use with a diaper laminating machine.

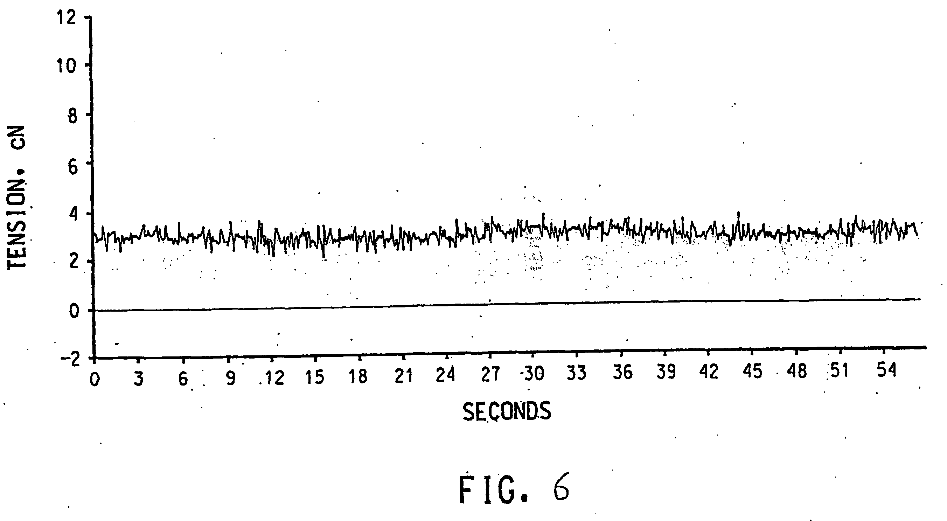

FIG. 6 is a graph of the relationship between the unwinding tension of Example 1 and time.
FIG. 7 is a histogram showing the coefficient of variation of the unwinding tension of Example 1.
FIG. 8 is a graph of the relationship between the unwinding tension of Comparison. Example 1 and time.
FIG. 9 is a histogram showing the coefficient of variation of the unwinding tension of Comparison Example 1.
FIG. 10 is a graph of the relationship between the unwinding tension of Example 2 and time.

## DETAILED DESCRIPTION OF THE INVENTION

As used herein, "elastomeric fiber" means a fiber which, free of diluents, has a break elongation in excess of 100% independent of any crimp and which when stretched to twice its length, held for one minute, and then released, retracts to less than 1.5 times its original length within one minute of being released. Such fibers include rubber fiber, spandex, elastomeric polyolefin fibers, and polyetherester fibers, and can be covered with non-elastomeric fibers or can be bare (uncovered). "Spandex" means a manufactured fiber in which the fiber-forming' substance is a long chain synthetic polymer comprised of at least 85% by weight of a segmented polyurethane. "Package relaxation" is the reduction in length of an elastomeric fiber that occurs when the fiber is removed from a wound package; when package relaxation is not uniform throughout the package, fiber diameter, elongation, and tension are also not uniform, and unless corrective measures are taken, undesirable nonuniformities can occur in the products made from these fibers.

"Compound roll" means a roll of the invention, comprising a plurality of undriven first rolls rotatably mounted on a second, driven roll in which the diameter of the second roll is larger than the diameter of each first roll, the axis of each first roll is substantially parallel to the axis of the second roll, and the axes of the plurality of first rolls are arranged substantially radially symmetrically about the axis of the second roll. "Radial symmetry" of the compound roll means that rotation of the roll about its longitudinal axis by 360/n degrees, in which 'n' is an integer representing the 'n-fold' symmetry of the roll, results in substantially the same end-on appearance as before rotation.

The compound roll of this invention (a 'yarn feeding apparatus') can assist in feeding at least one fiber to downstream equipment so that the tension in the fiber is low and uniform and package relaxation is corrected. For example, the compound roll is useful in unwinding fiber from a fiber package and feeding it to, for example, a diaper or sanitary napkin manufacturing line, a winder, a knitting machine, a coverer, a spinning machine, a twister, a loom, or other downstream equipment. The compound roll can be used with either rolling takeoff or over-end takeoff (OETO) and with one or more fibers.

The apparatus and method of the invention can be used with natural, semi-synthetic, or synthetic fibers and are particularly useful with bare elastomeric fibers. The package from which the fiber is removed can be a wound or piddled package.

The surface of many fibers, for example having a Roeder kinetic coefficient of friction of at least 0.05 and preferably greater than 0.5, is sufficient to cause' the first, small rolls of the compound roll to rotate as the fiber passes over them during use. The Roeder kinetic coefficient of friction can be measured by a method similar to that in Journal of the Society of Fiber Science and Technology, Japan (Sen-i Gakkaishi) Vol 41, No. 5, pages T211-212 (1985). In a modification of that method, a spandex sample having a weight on each end can be hung transversely across a matte-surface, 50-mm diameter, metal cylinder, with the weight on one end of the fiber fixed to the hook of a torsion balance. The cylinder can be rotated at 210 rpm and the position of the torsion balance arm adjusted to keep the hook of the balance at rest. The arm position can be read, and the coefficient of friction can be calculated from the following formula:

$$T_1 = T_0 exp(\mu\pi)$$

wherein $T_1$ is the weight on the spandex (for example 100g for a 620 decitex sample), $T_0$ is $T_1$ minus the torsion balance reading, and $\mu$ is the Roeder kinetic coefficient of friction. When measured by this method, 940 dtex Lycra® spandex T-127 XA has a calculated Roeder kinetic coefficient of friction of 1.84.

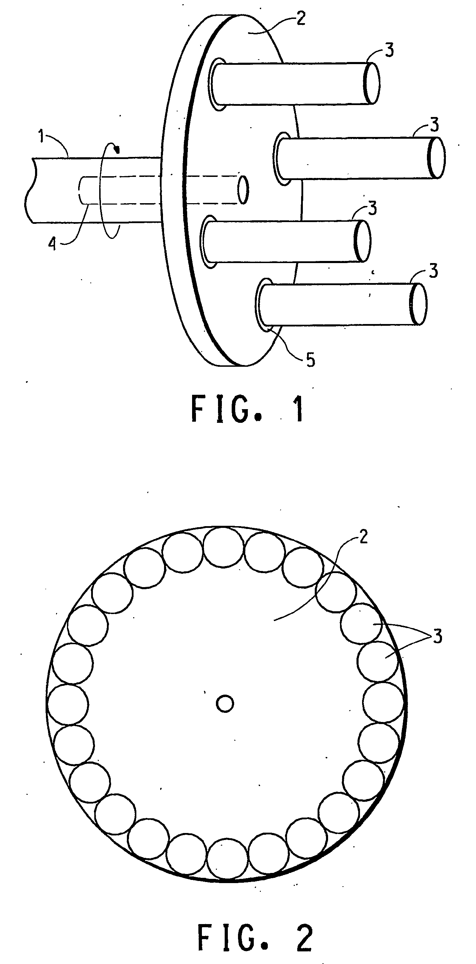

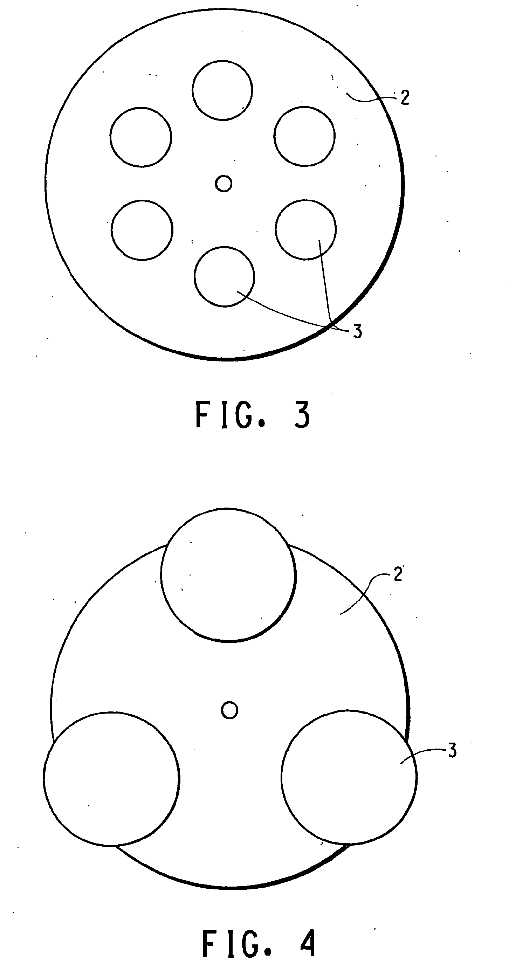

Referring first to Figure 1, the apparatus of the invention comprises a plurality of small rolls 3 freely rotatably mounted on a large driven roll 2, which can be in turn fixed to drive shaft 1 by fastening screw 4. Roll 2 can be a disk. Drive shaft 1 can be connected to a motor (not shown) or the drive unit of downstream equipment (not shown). The ratio of the diameter of each of small rolls 3 to that of large roll 2 can be about 0.01-0.045. The axis of each of the small rolls 3 is substantially parallel to that of the large roll 2, and, as can be seen in Figures 2, 3, and 4, the axes of the plurality of small rolls are substantially symmetrically radially arranged with respect to the axis of the large roll. Such symmetry can reduce undesirable vibrations during operation of the compound roll.

There can be 3-24 small rolls 3 mounted on large roll 2; for ease of manufacture and use, it is preferred that the number of small rolls be about 4-6. Small rolls 3 are freely rotatable, that is, rotatable in either direction and undriven, and can ride on bearings 5 so that they can rotate under the influence of friction from the passing fiber (not shown). The frictional characteristics of the circumferential surface of the small rolls can be adapted so that the fiber can rotate the small rolls as it passes over them without generating roll wraps.

The small rolls can be positioned so that portions of their circumferences are outside, inside, or coincident with that of the large roll. Figure 2 is a schematic end view showing an embodiment of the compound roll of the invention having twenty-four small rolls 3, a portion of whose circumferences are substantially tangential to the circumference of the large roll 2.

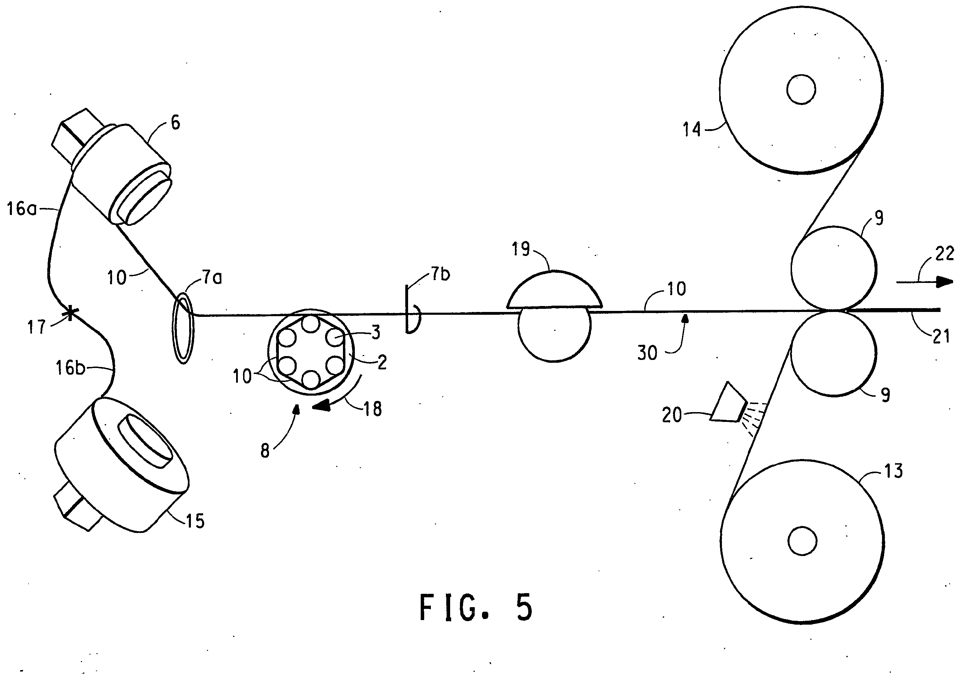

Figure 3 is a schematic end view showing another embodiment of the compound roll of the invention having six small rolls whose circumferences are within the circumference of the large roll. Figure 4 is a schematic end view showing an embodiment of the compound roll (yarn feeding apparatus) of the invention in which three small rolls 3 are mounted on a large roll so that portions of their circumferences are outside the circumference of the large roll.

To increase the rigidity of the apparatus and reduce twisting of the fiber, an additional large roll or disk can be attached to the previously free ends of the small rolls so that they are supported at both ends.

The rolls can be of metal (e.g., carbon steel, stainless steel, or titanium), plastic, ceramic or a composite and can be made wear-resistant by known methods. To reduce yarn breakage, it can be advantageous for the surfaces of the small rolls 3 to be configured so that the fibers passing over them do not cross each other, for example by providing circumferential grooves on the surfaces of the rolls. Such grooves can be especially useful in keeping fibers separate from each other when more than one package and fiber are used with each compound roll. In OETO from multiple packages, the small rolls 3 can be sufficiently long to prevent one fiber balloon from contacting an adjacent fiber balloon.

Utilizing the method of the invention, at least one fiber is passed around the plurality of first (small) rolls of the compound roll of the invention, optionally after being removed from at least one fiber package. The fiber has a coefficient of friction sufficiently high to rotate the first rolls as the fiber passes over them. The fiber is then fed at a predetermined speed to downstream equipment. Preferably, the second (large) roll of the compound roll is rotated at a circumferential ("surface") speed that is higher than the speed at which the downstream equipment uses the fiber, so that the fiber speed at the compound roll is higher than at the downstream equipment, for example by a ratio of 1.2:1 to 4.5:1. The "circumferential speed" of the large roll is the roll's surface speed at the radial distance from its axis at which the small rolls come into contact with the fiber, which speed therefore can be greater than, equal to, or less than, the linear speed of the physical periphery of the large roll, depending on the position of the small rolls on the large roll. The fiber speed at the compound roll can be about 30-900 m/min and can be somewhat different from the large roll circumferential speed due to the free rotation of the small rolls.

Figure 5 schematically illustrates an embodiment of a method of the invention using the compound roll of the invention, here in connection with a downstream operation in which elastomeric fiber 10 can be laminated between film 13 and nonwoven fabric 14 by nip rolls 9. In this embodiment, elastomeric fiber 10 is unwound from package 6, either with a rolling takeoff or, preferably, with an over-end takeoff. When an over-end takeoff is used, transfer tail 16a and lead end 16b can be used to connect first package 6 to second package 15 by knot or splice 17 for continuous use of the fiber. Compound roll 8, which can rotate in the direction of fiber travel (arrow 18), comprises a plurality of freely rotatable smaller first rolls 3 mounted on larger driven rotatable second roll 2. Fiber 10 is then passed at least once (preferably only once) around the entire group of small-diameter rolls 3. Fiber guides 7a and 7b can assist in positioning fiber 10; guide 7a in particular can control ballooning of the fiber as it is removed from the package. Fiber 10 does not pass completely around any one of small rolls 3, which can rotate under the influence of friction from fiber 10 passing thereover. To reduce fiber breakage, fiber 10 can pass around the group of rolls 3 without contacting itself or other fibers.

Optionally, tension device 19 can apply tension to fiber 10 by slowing its progress toward downstream equipment. Adhesive 20 can be applied before the three components enter nip rolls 9, and laminate 21 can exit in the direction of arrow 22, for example for subsequent use in making diapers. For smooth operation of the inventive method when fiber is being removed from a package, it is preferred that the distance between the package and the compound roll be greater than 1.5 times the package diameter. The preferred distance between the compound roll and the downstream tension rolls can depend on the speed of the fiber and can be selected so that the fiber does not sag.

In the Examples, the tension of the yarn leaving the compound roll a) was applied by nip rolls 9 (see Figure 5), b) was measured using an electronic tensiometer manufactured by the Rothschild Company at about the position indicated as "30", and c) is reported in centiNewtons or coefficient of variation, as indicated. The tension was typical of that used to feed a diaper-making machine. The distance between the package and the compound roll was 0.8 m, and that between the compound roll and the nip rolls was 0.5 m. Unless otherwise noted, the ratio of the circumferential speed of the compound roll to the downstream equipment use speed was 1.66.

In Examples 4-8 and Comparison Examples 2-6, the unwinding tensions of the package "outer layer" were measured at the surface of the package, those of the "middle layer" were measured about half-way through the package, and those of the inner layer were measured where a 5-mm layer of fiber remained on the package.

## EXAMPLE 1

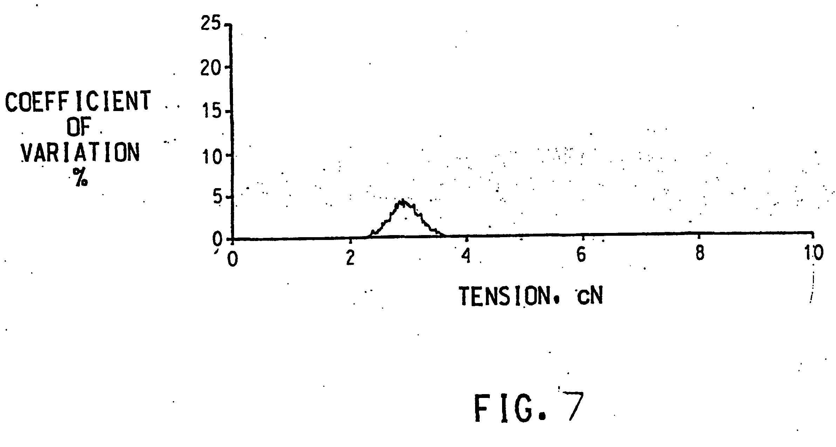

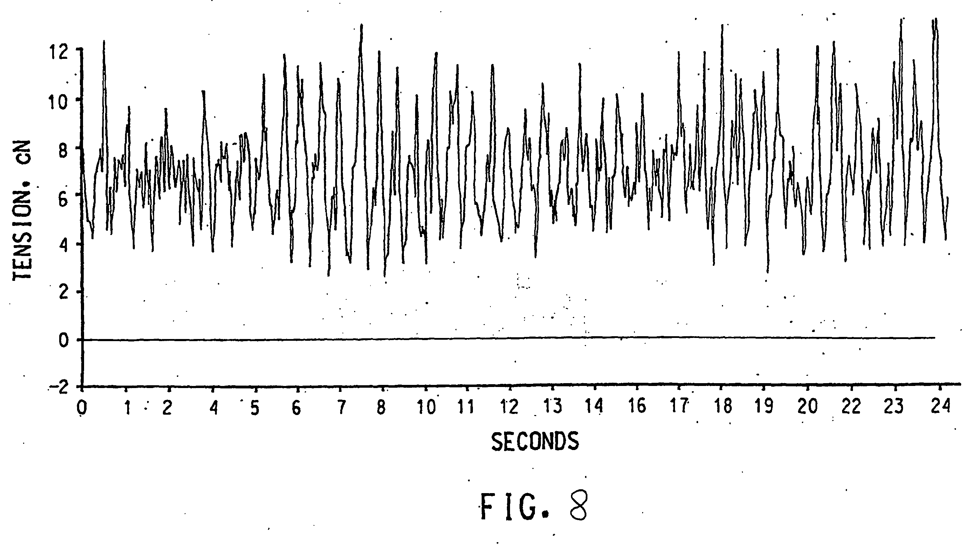

A 4.5-kg package of 620 decitex Lycra® T-127 spandex (a registered trademark of E. I. du Pont de Nemours and Company) to which no finish had been applied was unwound over-end using a stainless steel compound roll as shown in Figure 1 (200-mm large roll diameter, 2.0-mm small roll diameter). Variation in the unwinding tension of the package outer layer was measured at a fiber unwinding speed of 100 m/min. The unwinding tension results are plotted in FIG. 7, which shows a low level of tension and low-tension variability versus time. FIG. 8 is a histogram of the coefficient of variation of the tension showing that the coefficient of variation was very narrowly distributed.

## Comparison Example 1

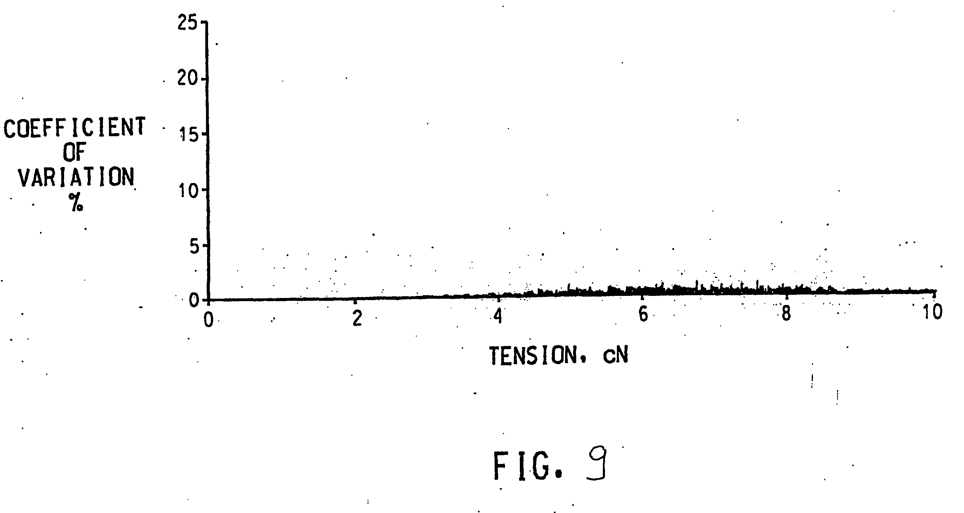

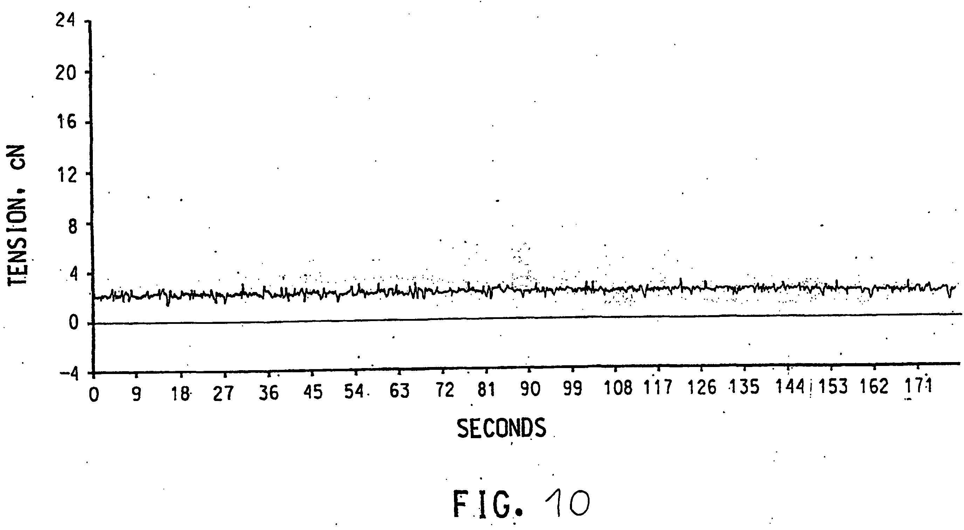

Example 1 was repeated, but without the compound roll of the invention. The unwinding tension results are plotted versus time in FIG. 9, which shows high tension and a wide range of tension values. FIG. 10 is a histogram of the coefficient of variation illustrating high variability when the compound roll was not used. Measurement was stopped after 24 seconds to protect the tensiometer.

## EXAMPLE 2

Example 1 was repeated, but at 200 m/min and with a ratio of the circumferential speed of the compound roll to the equipment use speed of 1.33. The unwinding tension results are plotted versus time in FIG. 11, which shows a very narrow range of tension values.

## EXAMPLE 3

Example 1 was repeated, but with a 3-kg package of spandex at 250 m/min. The unwinding tensions of the package outer layer, middle layer and inner layer were measured at a fiber unwinding speed of 250 m/min. The results are reported in Table 1, which gives the average tension, minimum tension, maximum tension and coefficient of variation of the tension within each layer. "Comp." indicates a Comparison Example.

In Examples 4, 5, 6 and 7, Example 3 was repeated with Lycra® T-127 spandex of various decitex and package size, and the test conditions and results are also given in Table 1.

## Comparison Example 2

Example 3 was repeated, but without the compound roll of the invention, and the results are summarized in Table 1.

In Comparison Examples 3, 4, 5, and 6, Comparison Example 2 was repeated with Lycra® T-127 of various decitex and package size, and the conditions and results are also given in Table 1.

**Table 1**

| Example | Package size, kg | Fiber decitex | Location In package | Average tension (cN) | Minimum tension (cN) | Maximum tension (cN) | Coeff. of variation (%) |
|---|---|---|---|---|---|---|---|
| 3 | 3 | 620 | outer | 3.0 | 3.1 | 4.9 | 6 |
| | | | middle | 2.1 | 1.3 | 2.9 | 10 |
| | | | inner | 2.0 | 1.1 | 2.9 | 12 |
| Comp. 2 | 3 | 620 | outer | 5.3 | 3.0 | 13.0 | 17 |
| | | | middle | 6.6 | 3.3 | 16.2 | 21 |
| | | | inner | 7.9 | 3.6 | 17.5 | 25 |
| 4 | 1 | 940 | outer | 2.1 | 3.0 | 5.4 | 7 |
| | | | middle | 2.0 | 0.7 | 2.9 | 13 |
| | | | inner | 2.2 | 1.2 | 3.2 | 11 |
| Comp.3 | 1 | 940 | outer | 3.0 | 1.7 | 6.1 | 16 |
| | | | middle | 5.3 | 1.9 | 13.8 | 24 |
| | | | inner | 6.7 | 2.2 | 17.7 | 25 |
| 5 | 4.5 | 940 | outer | 3.0 | 1.9 | 4.9 | 12 |
| | | | middle | 2.5 | 1.2 | 3.8 | 14 |
| | | | inner | 2.3 | 1.1 | 3.6 | 14 |

Table continued

| Example | Package size, kg | Fiber decitex | Location In package | Average tension (cN) | Minimum tension (cN) | Maximum tension (cN) | Coeff. of variation (%) |
|---|---|---|---|---|---|---|---|
| Comp. 4 | 4.5 | 940 | outer | 1.1 | -0.2 | 5.0 | 50 |
| | | | middle | 7.4 | 2.9 | 18.1 | 25 |
| | | | inner | 8.5 | 3.1 | 28.2 | 31 |
| 6 | 1 | 1240 | outer | 2.8 | 2.5 | 5.5 | 9 |
| | | | middle | 2.0 | 0.4 | 3.7 | 16 |
| | | | Inner | 2.4 | 1.2 | 3.8 | 13 |
| Comp. 5 | 1 | 1240 | outer | 5.0 | 2.7 | 11.2 | 18 |
| | | | middle | 7.0 | 2.9 | 16.6 | 23 |
| | | | Inner | 8.7 | 3.4 | 32.7 | 28 |
| 7 | 4.5 | 1240 | outer | 2.6 | 2.0 | 5.8 | 13 |
| | | | middle | 2.5 | 1.3 | 3.9 | 14 |
| | | | Inner | 2.4 | 1.0 | 6.4 | 15 |
| Comp.6 | 4.5 | 1240 | outer | 2.2 | 0.5 | 6.7 | 35 |
| | | | middle | 10.0 | 3.9 | 23.6 | 24 |
| | | | Inner | 12.5 | 6.1 | 34.0 | 59 |

Examination of the data in Table 1 reveals that when the compound roll of the invention was used, the takeoff tension was lower (especially for the middle and inner layers), and the coefficient of variation of the tension within each layer was lower, than when the roll was not used. The surface layer results for Comparison Examples 4 and 6 appear to be anomalous. In addition, the difference in tension between layers was lower, being on average 0.3 cN when the roll was used and 2.8 cN when the roll was not used, indicating significantly improved uniformity in package relaxation.

When disposable diapers were prepared from spandex unwound according to the Examples of the invention and fed to a diaper-making line, the diapers had even gathers, reflecting uniform spandex tension. When diapers were prepared from spandex unwound according to the Comparison Examples and fed to a diaper line, the diapers had uneven gathers, reflecting nonuniform spandex tension.

**Claims**

1. A compound roll (8) for feeding at least one fiber to downstream equipment, **characterised in that** said compound roll comprises a plurality of undriven first rolls (3) wherein each first roll (3) has an axis and is freely rotatably mounted on a second, driven rotatable roll (2) which has a diameter larger than the diameter of each first roll (3) and an axis, wherein the axis of each first roll (3) is substantially parallel to the axis of the second roll (2), and wherein the axes of the plurality of first rolls (3) are substantially radially symmetrically arranged around the axis of the second roll (2).

2. The compound roll (8) of claim 1 wherein the number of first rolls (3) is 4-6.

3. The compound roll (8) of claim 1 wherein the ratio of the diameter of each first roll (3) to the diameter of the second roll (2) is about 0.01 - 0.045.

4. The compound roll (8) of claim 1 wherein at least one first roll (3) has at least one circumferential groove.

5. A method for feeding at least one fiber (10) to downstream equipment, **characterised in that** said method comprises the steps of:

passing the fiber (10) around a compound roll (8) comprising a plurality of undriven first rolls (3), wherein each first roll (3) has an axis and is rotatably mounted on a second, driven rotatable roll (2) which has a diameter larger than the diameter of each first roll (3), and an axis, wherein the axis of each first roll (3) is substantially parallel to the axis of the second roll (2), and the axes of the plurality of first rolls (3) are substantially radially symmetrical around the axis of the second roll (2); and

feeding the fiber (10) at a predetermined speed to the downstream equipment;

wherein the fiber has a coefficient of friction sufficient to cause the first rolls (3) to rotate during use.

6. The method of claim 5 wherein the fiber (10) is an elastomeric fiber and the fiber speed at the compound roll is about 1.2 - 4.5 times the equipment use speed.

7. The method of claim 5 wherein the equipment is diaper-making equipment.

8. The method of claim 5 further comprising a step, before the fiber (10) is passed around the compound roll (8), of unwinding the fiber (10) from at least one package, and wherein the fiber (10) is an elastomeric fiber having a Roeder kinetic coefficient of friction of at least about 0.05, and the fiber speed at the compound roll (8) is about 30-900 m/min.

## Patentansprüche

1. Compound-Rolle (8) zum Zuführen mindestens einer Faser zu einer nachgeschalteten Anlage, **dadurch gekennzeichnet, dass** die Compound-Rolle eine Mehrzahl nicht angetriebener erster Rollen (3) aufweist, wobei jede erste Rolle (3) eine Achse hat und frei rotationsfähig auf einer zweiten, angetriebenen rotationsfähigen Rolle (2) aufgebaut ist, die einen größeren Durchmesser als den Durchmesser jeder ersten Rolle (3) und einer Achse hat, wobei die Achse jeder ersten Rolle (3) weitgehend parallel zu der Achse der zweiten Rolle (2) ist und wobei die Achsen der Mehrzahl erster Rollen (3) weitgehend radialsymmetrisch um die Achse der zweiten Rolle (2) angeordnet sind.

2. Compound-Rolle (8) nach Anspruch 1, wobei die Zahl der ersten Rollen (3) 4 bis 6 beträgt.

3. Compound-Rolle (8) nach Anspruch 1, wobei das Verhältnis des Durchmessers jeder ersten Rolle (3) zu dem Durchmesser der zweiten Rolle (2) etwa 0,01 bis 0,045 beträgt.

4. Compound-Rolle (8) nach Anspruch 1, wobei mindestens eine erste Rolle (3) mindestens eine umlaufende Rille hat.

5. Verfahren zum Zuführen mindestens einer Faser (10) zu einer nachgeschalteten Anlage, **dadurch gekennzeichnet, dass** das Verfahren die Schritte umfasst:

die Faser (10) um eine Compound-Rolle (8) führen, die eine Mehrzahl nicht angetriebener erster Rollen (3) aufweist, wobei jede erste Rolle (3) eine Achse hat und rotationsfähig auf einer zweiten, nicht angetriebenen rotationsfähigen Rolle (2) aufgebaut ist, die einen größeren Durchmesser als den Durchmesser jeder ersten Rolle (3) hat, und eine Achse, wobei die Achse jeder ersten Rolle (3) weitgehend parallel zu der Achse der zweiten Rolle (2) ist und die Achsen der Mehrzahl erster Rollen (3) weitgehend radialsymmetrisch um die Achse der zweiten Rolle (2) sind; und

Zuführen der Faser (10) mit einer vorbestimmten Geschwindigkeit zu der nachgeschalteten Anlage;

wobei die Faser einen Reibungskoeffizienten hat, der ausreichend ist, um die ersten Rollen (3) während des Gebrauchs zu drehen.

6. Verfahren nach Anspruch 5, bei welchem die Faser (10) eine elastomere Faser ist und die Fasergeschwindigkeit an der Compound-Rolle etwa das 1,2- bis 4,5-fache der Gebrauchsgeschwindigkeit der Anlage beträgt.

7. Verfahren nach Anspruch 5, bei welchem die Anlage eine Anlage zur Windelfertigung ist.

8. Verfahren nach Anspruch 5, ferner umfassend einen Schritt vor der Führung der Faser (10) um die Compound-Rolle (8) des Abwickelns der Faser (10) von mindestens einem Wickelkörper, und wobei die Faser (10) eine elastomere Faser ist, die einen kinetischen Reibungskoeffizienten nach Roeder von mindestens etwa 0,05 hat, und die Fasergeschwindigkeit an der Compound-Rolle (8) etwa 30 bis 900 m/min beträgt.

**Revendications**

1. Rouleau combiné (8) pour approvisionner au moins une fibre vers un équipement en aval, **caractérisé en ce que** ledit rouleau combiné comprend une pluralité de premiers rouleaux non actionnés (3), où chaque premier rouleau (3) possède un axe et est monté de façon à tourner librement sur un deuxième rouleau rotatif, actionné (2) qui possède un diamètre plus grand que le diamètre de chaque premier rouleau (3) et un axe, où l'axe de chaque premier rouleau (3) est substantiellement parallèle à l'axe du deuxième rouleau (2) et où les axes de la pluralité de premiers rouleaux (3) sont substantiellement arrangés radialement symétriquement autour de l'axe du deuxième rouleau (2).

2. Rouleau combiné (8) suivant la revendication 1, dans lequel le nombre de premiers rouleaux (3) est 4-6.

3. Rouleau combiné (8) suivant la revendication 1, dans lequel le rapport du diamètre de chaque premier rouleau (3) sur le diamètre du deuxième rouleau (2) est d'environ 0,01-0,045.

4. Rouleau combiné (8) suivant la revendication 1, dans lequel au moins un premier rouleau (3) possède au moins une rainure en circonférence.

5. Procédé pour approvisionner au moins une fibre (10) vers un équipement en aval, **caractérisé en ce que** ledit procédé comprend les étapes:

de passage de la fibre (10) autour d'un rouleau combiné (8) comprenant une pluralité de premiers rouleaux non actionnés (3), où chaque premier rouleau (3) possède un axe et est monté de façon à tourner sur un deuxième rouleau rotatif, actionné (2) qui possède un diamètre plus grand que le diamètre de chaque premier rouleau (3) et un axe, où l'axe de chaque premier rouleau (3) est substantiellement parallèle à l'axe du deuxième rouleau (2) et les axes de la pluralité de premiers rouleaux (3) sont substantiellement radialement symétriques autour de l'axe du deuxième rouleau (2); et
d'approvisionnement de la fibre (10) à une vitesse prédéterminée vers l'équipement en aval;
dans lequel la fibre présente un coefficient de frottement suffisant pour faire tourner les premiers rouleaux (3) pendant l'utilisation.

6. Procédé suivant la revendication 5, dans lequel la fibre (10) est une fibre élastomère et la vitesse de la fibre au niveau du rouleau combiné est environ 1,2-4,5 fois la vitesse d'utilisation de l'équipement.

7. Procédé suivant la revendication 5, dans lequel l'équipement est un équipement de fabrication de langes.

8. Procédé suivant la revendication 5, comprenant en outre une étape, avant le passage de la fibre (10) autour du rouleau combiné (8), de déroulement de la fibre (10) à partir d'au moins une bobine et dans lequel la fibre (10) est une fibre élastomère présentant un coefficient de frottement cinétique Roeder d'au moins environ 0,05, et la vitesse de la fibre au niveau du rouleau combiné (8) est d'environ 30-900 m/min.

FIG. 1

FIG. 2

FIG. 3

FIG. 4

FIG. 5

FIG. 6

EP 1 305 248 B1

FIG. 7

FIG. 8

EP 1 305 248 B1

FIG. 9

FIG. 10

EP 1 305 248 B1